# EUROPÄISCHE PATENTANMELDUNG

(11) **EP 4 699 627 A1**
(43) Veröffentlichungstag der Anmeldung: **25.02.2026**
(21) Anmeldenummer: 25196827.7
(22) Anmeldetag: 19.08.2025
(51) Int. Cl.: A61M 1/16, A61M 1/36

(54) **EXTRAKORPORALE BLUTBEHANDLUNGSMASCHINE UND VERFAHREN ZUM AUTOMATISCHEN PRIMEN EINES DIALYSATORS**

(30) Priorität: 20.08.2024 DE 102024123677
(71) Anmelder: B. Braun Avitum AG, 34212 Melsungen (DE)
(72) Erfinder: BROEKER, Bjoern, 34355 Staufenberg (DE)
(74) Vertreter: Winter, Brandl - Partnerschaft mbB

(57) **Zusammenfassung**

Die Offenbarung betrifft eine extrakorporale Blutbehandlungsmaschine mit einem Hohlfasern (2) aufweisenden Dialysator (19) mit einer Blutseite (4) und einer Dialysierflüssigkeitsseite (6), einem extrakorporalen Blutkreislauf und einem Dialysierflüssigkeitskreislauf. Die extrakorporale Blutbehandlungsmaschine weist eine Steuereinheit (48) auf, welche eingerichtet und vorbereitet ist, ein automatisches Primen der Blutbehandlungsmaschine derart durchzuführen, dass sie nacheinander oder alternierend einen Transmembrandruck (10) mit einem Druckgefälle von der Dialysierflüssigkeitsseite (6) zur Blutseite (4) und von der Blutseite (4) zur Dialysierflüssigkeitsseite (6) einstellt, um Luft beim Primen aus der semipermeablen Membran (8) zu entfernen. Dabei weist der Dialysierflüssigkeitskreislauf einen Dialysierflüssigkeitszulauf (30) mit einem Zulaufventil (32), einen Dialysierflüssigkeitsablauf (36) mit einem Ablaufventil (38) sowie eine Pumpe (42) auf, und die Steuereinheit (48) ist eingerichtet und vorbereitet, den Transmembrandruck (10) mit einem Druckgefälle von der Blutseite (4) zur Dialysierflüssigkeitsseite (6) durch ein Öffnen des Ablaufventils (38) und/oder durch ein Betätigen der Pumpe (42) einzustellen. Die Offenbarung betrifft weiterhin ein Verfahren zum Primen eines Hohlfasern (2) aufweisenden Dialysators (19) sowie ein Computerprogramm.

## Beschreibung

### Technisches Gebiet

Die vorliegende Offenbarung betrifft eine extrakorporale Blutbehandlungsmaschine, insbesondere eine Dialysemaschine, mit einem Hohlfasern aufweisenden Dialysator. Außerdem betrifft die Offenbarung ein Verfahren zum automatischen Primen eines Dialysators der extrakorporalen Blutbehandlungsmaschine.

### Hintergrund der Offenbarung

Auf dem Gebiet der extrakorporalen Blutbehandlung, insbesondere der Dialyse, ist es üblich, dass Patienten, deren Nieren nicht mehr in der Lage sind, Abfallprodukte aus dem Blut zu filtern, regelmäßig behandelt werden müssen. Diese Behandlungen erfordern den Einsatz von Dialysemaschinen, die das Blut des Patienten außerhalb des Körpers (extrakorporal) reinigen, indem sie es durch einen Dialysator leiten, in dem die Reinigung über eine semipermeable Membran stattfindet. Bekannte Systeme weisen typischerweise einen Hohlfaserdialysator auf, der eine Blutseite und eine Dialysierflüssigkeitsseite aufweist, die durch eine semipermeable Membran getrennt sind. Das Prinzip der Dialyse basiert auf dem Diffusionsprozess, bei dem Abfallstoffe und überschüssige Flüssigkeiten durch die Membran in eine Dialysierflüssigkeit (Dialysat) übergehen, während das gereinigte Blut zum Patienten zurückgeführt wird.

Ein wesentlicher Schritt vor Beginn jeder Dialyse ist das Priming bzw. Primen des Dialysators, d.h. das Vorbereiten und Füllen bzw. Spülen des Dialysators/ Filters mit einer speziellen Flüssigkeit, insbesondere einer Spülflüssigkeit/ Primingflüssigkeit, um Luftblasen aus dem Dialysator, insbesondere der Membran des Dialysators, zu entfernen. Luftblasen im Dialysator können zu ernsthaften Komplikationen führen, beispielsweise Luftembolien beim Patienten. Gemäß bekannter Verfahren wird das Priming bzw. Primen manuell oder halbautomatisch durchgeführt, indem Flüssigkeit durch den Dialysator und die Schlauchsysteme gepumpt wird oder durch einen manuellen Klopfvorgang durch den Bediener, um Luft zu verdrängen. Konventionelle Hohlfaserdialysatoren werden üblicherweise zunächst blutseitig gefüllt, bevor die Dialysierflüssigkeitsseite gefüllt wird. Dabei ist ein Drehen des Dialysators erforderlich, da während der Therapie die Flüssigkeitsströme auf beiden Seiten in üblicher Weise gegenläufig verlaufen. Auf diese Weise können Schadstoffe, Abfallprodukte sowie überschüssiges Wasser am besten aus dem Blut entfernt und mit der Dialysierflüssigkeit abtransportiert werden. Beispielsweise beschreibt die
EP 3 174 571 B1 eine gattungsgemäße extrakorporale Blutbehandlungsmaschine mit einem Hohlfaserdialysator.

Trotz der beträchtlichen Fortschritte auf dem Gebiet der Dialysetechnologie und der Automatisierung von Prozessen besteht nach wie vor ein Bedarf an verbesserten Methoden und Vorrichtungen, die ein effizienteres, sichereres und vor allem ein automatisiertes Priming, d. h. ohne einen zusätzlichen Drehvorgang des Dialysators durch den Operator, ermöglichen. Insbesondere besteht die Herausforderung darin, Luftblasen effektiv und zuverlässig aus der semipermeablen Membran des Dialysators zu entfernen, um das Risiko von Luftembolien zu minimieren und die Sicherheit des Patienten zu gewährleisten.

Allerdings liegen bei solchen Lösungen immer noch Grenzen hinsichtlich der Automatisierung des Priming-Prozesses und der Effektivität bei der Entfernung von Luftblasen aus der semipermeablen Membran des Dialysators vor.

### Kurzbeschreibung der vorliegenden Offenbarung

Daher liegt der vorliegenden Offenbarung die Aufgabe zugrunde, die vorstehend beschriebenen Nachteile zu vermeiden oder wenigstens abzumildern und insbesondere eine extrakorporale Blutbehandlungsmaschine bereitzustellen, welche eine möglichst vollständige Entfernung von Luft bzw. Luftblasen während eines Priming-Vorgangs effizient und insbesondere automatisiert ermöglicht.

Diese Aufgabe wird durch eine extrakorporale Blutbehandlungsmaschine gemäß den Merkmalen des Anspruchs 1, durch ein Verfahren zum automatischen Primen eines Dialysators einer extrakorporalen Blutbehandlungsmaschine gemäß den Merkmalen des Anspruchs 7 und durch ein Computerprogramm gemäß den Merkmalen des Anspruchs 14 gelöst. Vorteilhafte Ausführungsformen sind in den Unteransprüchen beansprucht und/oder werden nachfolgend erläutert.

Die Offenbarung betrifft demzufolge zunächst eine extrakorporale Blutbehandlungsmaschine, insbesondere eine Dialysemaschine, welche einen Hohlfasern/ Kapillaren aufweisenden Dialysator/ Filter mit einer Blutseite/ Blutkammer und einer Dialysierflüssigkeitsseite/ Dialysierflüssigkeitskammer, die voneinander durch eine semipermeable (halbdurchlässige) Membran getrennt sind, einen extrakorporalen Blutkreislauf, welcher über einen ersten blutseitigen Anschluss und einen zweiten blutseitigen Anschluss des Dialysators durch die Blutseite des Dialysators verläuft, und einen Dialysierflüssigkeitskreislauf, welcher über einen ersten dialysierflüssigkeitsseitigen Anschluss und einen zweiten dialysierflüssigkeitsseitigen Anschluss des Dialysators durch die Dialysierflüssigkeitsseite des Dialysators verläuft, aufweist. Dabei weist die extrakorporale Blutbehandlungsmaschine eine Steuereinheit auf, welche eingerichtet und vorbereitet ist, ein automatisches Primen/ Vorbereiten der Blutbehandlungsmaschine derart durchzuführen, dass sie nacheinander oder alternierend einen Transmembrandruck mit einem Druckgefälle von der Dialysierflüssigkeitsseite zur Blutseite und von der Blutseite zur Dialysierflüssigkeitsseite einstellt, um Luft, insbesondere Luftblasen, beim Primen aus der semipermeablen Membran zu entfernen/ abzuführen/ zu fördern/ auszuspülen.

Der Dialysierflüssigkeitskreislauf weist ferner einen Dialysierflüssigkeitszulauf mit einem Zulaufventil, einen Dialysierflüssigkeitsablauf mit einem Ablaufventil sowie eine Pumpe, insbesondere eine Ultrafiltrationspumpe, auf. Die Steuereinheit ist derart eingerichtet und vorbereitet, dass sie, insbesondere ausgehend von einer Bypass-Phase, in welcher in vorteilhafter Weise identische Drücke auf der Blutseite und der Dialysierflüssigkeitsseite des Dialysators vorliegen, den Transmembrandruck mit einem Druckgefälle von der Blutseite zur Dialysierflüssigkeitsseite über ein Betätigen der Pumpe und/oder durch ein Öffnen des Ablaufventils einstellt. Beispielsweise kann ein Unterdruck auf der Dialysierflüssigkeitsseite mithilfe der Ultrafiltrationspumpe und/oder durch das Öffnen des Ablaufventils erzeugt werden, wodurch ein solcher Transmembrandruck mit einem Druckgefälle von der Blutseite zur Dialysierflüssigkeitsseite einstellbar ist.

In anderen Worten ausgedrückt bildet ein Hohlfaser-Dialysator das zentrale Element der extrakorporalen Blutbehandlungsmaschine. Dieser Dialysator ist in zwei Bereiche unterteilt, die Blutseite und die Dialysierflüssigkeitsseite. Beide Seiten sind durch eine semipermeable Membran voneinander getrennt. Diese Anordnung ermöglicht den Austausch von Stoffen zwischen Blut und Dialysierflüssigkeit, beispielsweise Wasser von der Blutseite zur Dialysierflüssigkeitsseite, während gleichzeitig verhindert wird, dass Blutzellen und größere Moleküle die Membran passieren. Der Austausch zwischen diesen zwei Seiten erfolgt durch den osmotischen Austausch über die semipermeable Membran. Ein Transmembrandruck, d. h. eine Druckdifferenz zwischen den durch die Membran separierten Seiten ist durch eine Steuereinheit der extrakorporalen Blutbehandlungsmaschine einstellbar. Dieser Transmembrandruck weist nacheinander oder alternierend ein Druckgefälle von der Dialysierflüssigkeitsseite zur Blutseite und ein Druckgefälle von der Blutseite zur Dialysierflüssigkeitsseite auf. Die Steuereinheit ist also eingerichtet, den Transmembrandruck mit einem Druckgefälle von der Dialysierflüssigkeitsseite zur Blutseite einzustellen, und den Transmembrandruck mit einem Druckgefälle von der Blutseite zur Dialysierflüssigkeitsseite einzustellen, und zwar nacheinander oder alternierend.

Eine derartige Anordnung ermöglicht einen effizienten Stoffaustausch, indem sie zwei separate Kreisläufe für Blut und Dialysierflüssigkeit nutzt, die jeweils über spezifische Anschlüsse am Dialysator verlaufen. Durch die Implementierung einer Steuereinheit, die speziell dafür eingerichtet und vorbereitet ist, ein automatisches Primen durchzuführen, wird das Entfernen von Luft oder Luftblasen aus der semipermeablen Membran vor der eigentlichen Behandlung vereinfacht, indem ein Transmembrandruck nacheinander mit einem Druckgefälle von der Dialysierflüssigkeitsseite zur Blutseite und umgekehrt oder abwechselnd in beide Richtungen eingestellt wird. Diese technische Lösung adressiert die spezifische Herausforderung, Luftansammlungen im Dialysator effektiv zu beseitigen, die sonst die Leistungsfähigkeit der Dialyse beeinträchtigen oder gar Patientensicherheitsrisiken darstellen könnten. Insbesondere betrifft es das Entfernen von Luftblasen aus den Poren der nahezu schwammartigen Membran des Dialysators. Diese Luftblasen können durch das nacheinander gefolgte oder alternierende Einstellen der Transmembrandrücke und der dadurch erzeugten gerichteten Durchströmungen, vorzugsweise in Richtung einer Ultrafiltration und einer Rückfiltration, ausgespült werden. Besonders vorteilhaft ist es, wenn der Transmembrandruck mit einem Druckgefälle von der Dialysierflüssigkeitsseite zur Blutseite über die Steuereinheit eingestellt wird, da innerhalb der Hohlfasern, also auf der Blutseite des Dialysators, eine höhere Strömungsgeschwindigkeit erzeugbar ist, so dass die Luftblasen besser abgeführt werden können. Das abwechselnde bzw. alternierende bzw. nacheinander gefolgte Einstellen eines Transmembrandrucks mit einem Druckgefälle von der Dialysierflüssigkeitsseite zur Blutseite und eines Transmembrandrucks mit einem Druckgefälle von der Blutseite zur Dialysierflüssigkeitsseite ist ganz besonders vorteilhaft, da die Luftblasen dann durch Druckstöße in unterschiedliche Richtungen aus der Hohlfasermembran des Dialysators entfernt bzw. abtransportiert werden. Durch die Automatisierung des Priming-Prozesses kann die Maschine schnell und effizient auf Betriebsbereitschaft gebracht werden, was nicht nur die Handhabung für das medizinische Personal vereinfacht, sondern auch die Sicherheit und den Komfort für den Patienten erhöht. Insbesondere, ist ein manuelles Klopfen des Dialysators vor der eigentlichen Therapie nicht mehr notwendig, um eventuelle Luftblasen aus der Membran des Dialysators zu entfernen.

Offenbarungsgemäße wird eine präzise Kontrolle und Anpassung des Transmembrandrucks ermöglicht, was für die Effizienz der Dialysebehandlung von Bedeutung ist. Durch die Fähigkeit der Steuereinheit, den Transmembrandruck gezielt über diese Komponente(n) (Pumpe und/oder Ablaufventil) einzustellen, wird eine optimierte Entfernung von Luft, insbesondere Luftblasen, aus der semipermeablen Membran während des Priming-Prozesses ermöglicht, und zwar durch eine gerichtete Durchströmung der Membran des Dialysators, was die Sicherheit und Effektivität der gesamten Blutbehandlung erhöht.

In einer weiteren bevorzugten Ausführungsform der Offenbarung kann der extrakorporale Blutkreislauf einen arteriellen Blutschlauchabschnitt mit einer Blutpumpe und einen venösen Blutschlauchabschnitt mit einer venösen Absperreinrichtung, beispielsweise einem venösen Absperrventil oder einer venösen (Schlauch-)Klemme aufweisen, und die Steuereinheit kann eingerichtet und vorbereitet sein, den Transmembrandruck mit einem Druckgefälle von der Blutseite zur Dialysierflüssigkeitsseite über die Blutpumpe und/oder ein Schließen der venösen Absperreinrichtung einzustellen, und/oder den Transmembrandruck mit einem Druckgefälle von der Dialysierflüssigkeitsseite zur Blutseite durch ein (nachfolgendes) Öffnen der venösen Absperreinrichtung einzustellen. Beispielsweise kann die Steuereinheit eingerichtet sein, zum Erzeugen eines Druckstoßes auf der Blutseite bei laufender Blutpumpe ein kurzzeitiges Schließen und nachfolgendes Öffnen des venösen Absperrventils/ der venösen Klemme durchzuführen.

Somit kann beispielsweise ein Überdruck auf der Blutseite des Dialysators mithilfe der Blutpumpe und/oder ein Unterdruck auf der Dialysierflüssigkeitsseite durch ein Öffnen des Ablaufventils des Dialysierflüssigkeitskreislaufs erzeugt werden, wodurch ein Transmembrandruck mit einem Druckgefälle von der Blutseite zur Dialysierflüssigkeitsseite einstellbar ist.

Durch die Möglichkeit, den Transmembrandruck sowohl über die Blutpumpe als auch über das Ablaufventil zu regulieren, erhält die Steuereinheit eine flexible und präzise Methode, um optimale Bedingungen für das automatische Entfernen von Luftblasen zu schaffen und gleichzeitig eine effektive Dialysebehandlung zu gewährleisten.

Konventionelle Hohlfaserdialysatoren werden üblicherweise zunächst blutseitig gefüllt, bevor die Dialysierflüssigkeitsseite gefüllt wird. Hierfür ist dann auf der Dialysierflüssigkeitsseite ein Unterdruck über die (Ultrafiltrations-)Pumpe einstellbar, sodass die Priming-Flüssigkeit von der Blutseite zur Dialysierflüssigkeitsseite angezogen wird, um die Leitungen auf der Dialysierflüssigkeitsseite ebenfalls mit der Priming-Flüssigkeit zu füllen. Eine optionale Flussrichtungsumkehr der Priming-Flüssigkeit kann dann über die Druckstöße erfolgen, insbesondere durch das wechselseitige Öffnen bzw. Schließen der venösen Klemme bzw. des Ablaufventils auf der Dialysierflüssigkeitsseite.

Besonders bevorzugt kann die Steuereinheit eingerichtet sein, den Transmembrandruck mit einem Druckgefälle von der Dialysierflüssigkeitsseite zur Blutseite und den Transmembrandruck von der Blutseite zur Dialysierflüssigkeitsseite alternierend/ abwechselnd einzustellen.

Anders ausgedrückt können also die Transmembrandrücke mit einem Druckgefälle von der Blutseite zur Dialysierflüssigkeitsseite und von der Dialysierflüssigkeitsseite zur Blutseite nacheinander und wechselweise von der Steuereinheit eingestellt werden, und zwar mithilfe von Kombinationen der oben beschriebenen Möglichkeiten, die Transmembrandrücke einzustellen, d. h. über die (Ultrafiltrations-)pumpe und/oder das Ablaufventil und zusätzlich über die Blutpumpe und/oder die venöse Absperreinrichtung, insbesondere das venöse Absperrventil oder die venösen Klemme.

In einer besonders vorteilhaften Ausführungsform der vorliegenden Offenbarung kann der Dialysierflüssigkeitskreislauf einen Bypass-Pfad mit einem Bypass-Ventil aufweisen, über welches ein Bypass-Zustand der extrakorporalen Blutbehandlungsmaschine einstellbar ist, und die Steuereinheit kann eingerichtet und vorbereitet sein, ausgehend von diesem Bypass-Zustand, in welchem in vorteilhafter Weise identische Drücke auf der Blutseite und der Dialysierflüssigkeitsseite des Dialysators vorliegen, den Transmembrandruck mit einem Druckgefälle von der Dialysierflüssigkeitsseite zur Blutseite und von der Blutseite zur Dialysierflüssigkeitsseite durch ein gegensätzliches Öffnen und Schließen der (blutkreislaufseitigen) venösen Absperreinrichtung sowie des (dialysierflüssigkeitsseitigen) Ablaufventils alternierend einzustellen. Anders ausgedrückt wird die alternierende Einstellung der Druckgefälle auf beiden Seiten des Dialysators durch ein abwechselndes Öffnen der venösen Absperreinrichtung bei einem gleichzeitig geschlossenen Ablaufventil und des Ablaufventils bei einer gleichzeitig geschlossenen venösen Absperreinrichtung erzielt.

Diese alternierende Einstellung des Transmembrandrucks ist von besonderer Bedeutung, da sie eine besonders effiziente Entfernung von Luft, insbesondere von Luftblasen, aus der semipermeablen Membran während des Priming-Prozesses ermöglicht. Durch das alternierende Anlegen der unterschiedlichen Druckgefälle kann effektiver sichergestellt werden, dass sich Luftblasen, die sich möglicherweise auf einer Seite der Membran festgesetzt haben, durch den Wechsel des Druckgefälles zur gegenüberliegenden Seite bewegen und somit effektiver aus der Membran entfernt werden. Dieser Mechanismus der alternierenden Druckeinstellung verbessert nicht nur die Sicherheit und Effizienz des Priming-Prozesses, sondern trägt auch dazu bei, die Leistungsfähigkeit der Dialysebehandlung insgesamt zu steigern, da eine saubere und luftfreie Membran für einen optimalen Stoffaustausch zwischen Blut und Dialysierflüssigkeit sorgt. Beispielsweise kann nach einem Durchströmen von der Dialysierflüssigkeitsseite zur Blutseite, und der sich daraus erzeugten Abfuhr von Luftblasen in eine Richtung, eine entgegengesetzte Filtration eingestellt werden, die z.B. einem späteren Therapiefall entspricht, um hierdurch weitere Luftblasen aus der äußeren Dialysierflüssigkeitsseite der Membran zu entfernen.

Vorzugsweise ist die Steuereinheit der extrakorporalen Blutbehandlungsmaschine zusätzlich eingerichtet, den Transmembrandruck mit einem Druckgefälle von der Dialysierflüssigkeitsseite zur Blutseite und/oder von der Blutseite zur Dialysierflüssigkeitsseite jeweils pulsierend einzustellen, um Druckstöße/ Druckwellen entsprechend von der Dialysierflüssigkeitsseite zur Blutseite und/oder von der Blutseite zur Dialysierflüssigkeitsseite zu erzeugen. Anders ausgedrückt kann die Steuereinheit die weiter oben beschriebenen Komponenten zur Erzeugung der entsprechend benötigten Druckgefälle pulsartig, also in kurzen Abständen voneinander, ansteuern. Dadurch werden die Druckgefälle, vorzugsweise ebenfalls alternierend, pulsartig erzeugt, sodass Druckstöße in der (Priming-)Flüssigkeit erzeugt werden, und zwar auf der Blutseite und/oder auf der Dialysierflüssigkeitsseite.

Die durch die pulsierende Einstellung des Transmembrandrucks erzeugten Druckwellen oder Druckstöße, die entweder von der Dialysierflüssigkeitsseite zur Blutseite oder von der Blutseite zur Dialysierflüssigkeitsseite gerichtet sein können, ermöglichen eine effizientere Entfernung von Luft oder insbesondere Luftblasen aus der semipermeablen Membran während des Priming-Prozesses der Maschine. Die pulsierende Anpassung des Transmembrandrucks, die durch die Steuereinheit durchgeführt wird, fördert eine dynamischere Interaktion zwischen den beiden Seiten des Dialysators. Dies hat den Vorteil, dass die Entfernung von Luftblasen nicht nur durch ein konstantes Druckgefälle erfolgt, sondern durch eine Reihe von Druckstößen unterstützt wird.

Gemäß einer besonders vorteilhaften Ausführungsform der Offenbarung kann der Dialysierflüssigkeitskreislauf der extrakorporalen Blutbehandlungsmaschine zumindest zwei Zusatz-Strömungspfade mit jeweils einem Zusatzventil aufweisen, über welche der Dialysierflüssigkeitszulauf und der Dialysierflüssigkeitsablauf des Dialysierflüssigkeitskreislaufs fluidisch verbindbar bzw. trennbar sind. Vorzugsweise kann die Steuereinheit eingerichtet sein, die Zusatzventile derart zu steuern, dass eine Flussrichtung auf der Dialysierflüssigkeitsseite umkehrbar ist. Anders ausgedrückt sind zumindest zwei weitere Strömungspfade als fluidische Verbindungen des Dialysierflüssigkeitszulaufs und Dialysierflüssigkeitsablaufs auf Seite des Dialysierflüssigkeitskreislaufes angeordnet. Durch ein jeweiliges Öffnen bzw. Schließen der Zusatzventile der Zusatz-Strömungspfade kann dann das Zulauf- bzw. Ablaufventil des Dialysierflüssigkeitszulaufs umgangen werden, wodurch die Flussrichtung der (Priming-)Flüssigkeit auf der Dialysierflüssigkeitsseite umkehrbar ist. Lediglich beispielhaft wird auf die zum Anmeldetag der vorliegenden Patentanmeldung noch nicht veröffentlichte deutsche Patentanmeldung mit dem amtlichen Aktenzeichen 10 2025 114 056.1 verwiesen, welche einen ersten Zusatz-Strömungspfad mit einem Zusatzventil in Form eines 3/2-Wegeventils mit drei Anschlüssen und zwei Schaltstellungen, sowie einen zweiten Zusatz-Strömungspfad mit einem (passiven) Rückschlagventil aufweist, wobei ein Dialysierflüssigkeitszulauf und ein Dialysierflüssigkeitsablauf über die beiden Zusatzströmungspfade fluidisch verbindbar sind. In diesem Fall müsste lediglich das Zulaufventil, das Ablaufventil und das (einzige) Zusatzventil in Form des 3/2-Wegeventils angesteuert werden, um die Flussrichtung auf der Dialysierflüssigkeitsseite zu ändern bzw. umzukehren. Es müssen in anderen Worten nicht zwei Zusatzventile angesteuert werden.

Dadurch kann vorzugsweise bewirkt werden, dass die (Priming-)Flüssigkeiten innerhalb und außerhalb der Hohlfasern, also auf beiden Seiten der semipermeablen Membran, während des Priming- bzw. Befüll- bzw. Spülvorgangs des Dialysators in gleicher Richtung strömen können. Besonders vorteilhaft ist es, wenn diese gleichgerichtete Flussrichtung der Flüssigkeiten entgegen der Schwerkraft erfolgt, da diese der natürlichen Fließrichtung der leichteren Luftblasen im Dialysator entsprechen. Anders ausgedrückt entspricht eine derartige Flussrichtung der (Priming-)Flüssigkeit (auf der Blut- und der Dialysierflüssigkeitsseite) der Richtung, in welche die Luftblasen natürlich entweichen würden. Für eine nachfolgende Dialysetherapie können die Zusatzventile einfach entsprechend geschlossen werden, um für die Behandlung vorteilhafte entgegengesetzte Flussrichtungen auf der Blutseite und der Dialysierflüssigkeitsseite automatisch einzustellen. Ein manuelles (Um-)Drehen des Dialysators ist demnach nicht notwendig. Vorzugsweise ist hierfür der arterielle Blutschlauchabschnitt des extrakorporalen Blutkreislaufs und der Dialysierflüssigkeitsablauf des Dialysierflüssigkeitskreislaufs auf einer, vorzugsweise dem Boden zugewandten Seite, des Dialysators und entsprechend der venöse Blutschlauchabschnitt des extrakorporalen Blutkreislaufs und der Dialysierflüssigkeitszulauf des Dialysierflüssigkeitskreislaufs auf der anderen Seite des Dialysators angeordnet.

In einem weiteren vorteilhaften Aspekt der Offenbarung kann die Steuereinheit der extrakorporalen Blutbehandlungsmaschine eingerichtet sein, eine Förderrate der Pumpe/Förderpumpe derart zu erhöhen, dass eine turbulente Strömung auf der Dialysierflüssigkeitsseite des Dialysators erzeugt wird.

In anderen Worten ausgedrückt, kann die Förderrate der in dem Dialysierflüssigkeitskreislauf angeordneten Pumpe über einen vorbestimmten Schwellenwert/ Grenzwert eingestellt werden, welcher hoch genug ist, um eine anfangs laminare Strömung der (Priming-)Flüssigkeit außerhalb der Hohlfasern, d. h. auf der Dialysierflüssigkeitsseite von der Membran, in eine turbulente Strömung umzuwandeln.

Durch die Erhöhung der Förderrate der Pumpe, insbesondere der Ultrafiltrationspumpe, und die dadurch induzierte turbulente Strömung außerhalb der Hohlfaser wird eine effizientere Entfernung von Luft, insbesondere Luftblasen, aus der semipermeablen Membran während des Priming-Prozesses erreicht. Eine derartige turbulente Strömung bildet dünnere Randschichten im Kontaktbereich der Flüssigkeit und der Innenwand der Hohlfaser, was zu einem gleichmäßigeren Strömungsverlauf der Flüssigkeit führt. Dadurch ist die Bewegung von Luftblasen zur Oberfläche und deren anschließende Entfernung begünstigt. Somit ist eine gleichmäßigere und effektivere Luftblasenentfernung sichergestellt.

Gemäß einer weiteren vorteilhaften Ausführungsform der Offenbarung kann die Steuereinheit der extrakorporalen Blutbehandlungsmaschine eingerichtet sein, eine Förderrate der Blutpumpe derart zu erhöhen, dass eine turbulente Strömung auf der Blutseite des Dialysators erzeugt wird.

In anderen Worten ausgedrückt, kann die Förderrate der in dem Blutkreislauf angeordneten Blutpumpe über einen vorbestimmten Schwellenwert/ Grenzwert eingestellt werden, welcher hoch genug ist, um eine anfangs laminare Strömung der (Priming-)Flüssigkeit innerhalb der Hohlfasern, d. h. auf der Blutseite von der Membran, in eine turbulente Strömung umzuwandeln.

Die Erzeugung einer turbulenten Strömung hat den entscheidenden Vorteil, dass sie die Effizienz des Priming-Prozesses, also des Vorbereitungsprozesses der Maschine vor der eigentlichen Blutbehandlung signifikant steigert. Durch die turbulente Strömung wird gewährleistet, dass Luft, insbesondere Luftblasen, effektiver aus der semipermeablen Membran entfernt werden können. Dies ist darauf zurückzuführen, dass die turbulente Strömung, wie schon oben beschrieben, einen gleichmäßigeren Strömungsverlauf der (Priming-)Flüssigkeit gewährleistet.

Die Offenbarung betrifft weiterhin ein Verfahren zum automatischen Primen eines Hohlfasern aufweisenden Dialysators einer extrakorporalen Blutbehandlungsmaschine, mit einer Blutseite und einer Dialysierflüssigkeitsseite, welche voneinander durch eine semipermeable Membran getrennt sind. Das Verfahren weist dabei die Schritte Befüllen der Blutseite mit einer Flüssigkeit, insbesondere Priming-Flüssigkeit; Befüllen der Dialysierflüssigkeitsseite mit der Flüssigkeit; Einstellen eines Transmembrandrucks mit einem Druckgefälle von der Dialysierflüssigkeitsseite zur Blutseite des Dialysators über eine Steuereinheit, und Einstellen eines Transmembrandrucks mit einem Druckgefälle von der Blutseite zur Dialysierflüssigkeitsseite des Dialysators über die Steuereinheit durch ein Öffnen eines Ablaufventils und/oder ein Starten einer Pumpe in einem Dialysierflüssigkeitskreislauf auf, um Luft, insbesondere Luftblasen, beim Primen des Dialysators aus der semipermeablen Membran zu entfernen. Die Schritte Einstellen eines Transmembrandrucks mit einem Druckgefälle von der Dialysierflüssigkeitsseite zur Blutseite des Dialysators und Einstellen eines Transmembrandrucks mit einem Druckgefälle von der Blutseite zur Dialysierflüssigkeitsseite des Dialysators werden dabei nacheinander durchgeführt oder alternierend wiederholt. Dabei kann die Reihenfolge auch umgekehrt werden.

Vorzugsweise erfolgt der Schritt Einstellen des Transmembrandrucks mit dem Druckgefälle von der Dialysierflüssigkeitsseite zur Blutseite durch ein Öffnen einer venösen Absperreinrichtung im extrakorporalen Blutkreislauf, um entsprechend einen Überdruck auf der Dialysierflüssigkeitsseite (in Relation zur Blutseite) zu erzeugen.

Weiter bevorzugt weist das Verfahren einen zusätzlichen Schritt Einstellen einer Förderrate der Pumpe über die Steuereinheit, sodass eine turbulente Strömung auf der Dialysierflüssigkeitsseite des Dialysators erzeugt wird, auf.

**In** einer weiteren vorteilhaften Ausführungsform wird der Schritt Einstellen des Transmembrandrucks mit dem Druckgefälle von der Blutseite zur Dialysierflüssigkeitsseite des Dialysators durch ein Starten zumindest einer Blutpumpe und/oder ein Schließen der venösen Absperreinrichtung im extrakorporalen Blutkreislauf und durch ein Öffnen des Ablaufventils und/oder ein Starten der Ultrafiltrationspumpe im Dialysierflüssigkeitskreislauf durchgeführt, um entsprechend einen Überdruck auf der Blutseite und/oder einen Unterdruck auf der Dialysierflüssigkeitsseite zu erzeugen.

Weiter bevorzugt weist das Verfahren einen zusätzlichen Schritt Einstellen einer Förderrate der Blutpumpe über die Steuereinheit auf, sodass eine turbulente Strömung auf der Blutseite des Dialysators erzeugt wird.

**In** einer besonders vorteilhaften Ausführungsform des Verfahrens werden ausgehend von einem Bypass-Zustand der extrakorporalen Blutbehandlungsmaschine, in welchem bevorzugt blutseitig und dialysierflüssigkeitsseitig identische Drücke im Dialysator vorliegen, die Schritte Einstellen des Transmembrandrucks mit einem Druckgefälle von der Dialysierflüssigkeitsseite zur Blutseite und Einstellen des Transmembrandrucks mit einem Druckgefälle von der Blutseite zur Dialysierflüssigkeitsseite durch ein gegensätzliches Öffnen und Schließen einer venösen Absperreinrichtung im extrakorporalen Blutkreislauf sowie eines Ablaufventils im Dialysierflüssigkeitskreislauf alternierend wiederholt werden.

Beispielsweise kann die Steuereinheit eingerichtet sein, ausgehend von dem Bypass-Zustand identischer Drücke einen Transmembrandruck mit einem Druckgefälle von der Blutseite zur Dialysierflüssigkeitsseite einzustellen, dann wieder den Bypass-Zustand identischer Drücke einzustellen, um schließlich im Anschluss einen Transmembrandruck mit einem Druckgefälle von der Dialysierflüssigkeitsseite zur Blutseite einzustellen. Danach kann wieder der Bypass-Zustand identischer Drücke eingestellt werden, ausgehend von welchem der voranstehend beschriebene Vorgang gegebenenfalls erneut durchgeführt werden kann.

**In** einem besonders vorteilhaften Aspekt des Verfahrens können die Schritte Einstellen eines Transmembrandrucks mit einem Druckgefälle von der Dialysierflüssigkeitsseite zur Blutseite des Dialysators und Einstellen eines Transmembrandrucks mit einem Druckgefälle von der Blutseite zur Dialysierflüssigkeitsseite des Dialysators alternierend/ abwechselnd wiederholt werden. Vorzugsweise können die Schritte Einstellen des Transmembrandrucks mit einem Druckgefälle von der Dialysierflüssigkeitsseite zur Blutseite des Dialysators und Einstellen des Transmembrandrucks mit einem Druckgefälle von der Blutseite zur Dialysierflüssigkeitsseite des Dialysators jeweils pulsierend eingestellt werden, um Druckwellen/Druckstöße von der Dialysierflüssigkeitsseite zur Blutseite und/oder von der Blutseite zur Dialysierflüssigkeitsseite zu erzeugen.

**In** einem weiteren bevorzugten Aspekt weist das Verfahren einen zusätzlichen Schritt Umkehren einer Flussrichtung auf der Dialysierflüssigkeitsseite auf, vorzugsweise durch ein Einstellen, insbesondere durch das Öffnen, von jeweils einem Zusatzventil in zwei Zusatz-Strömungspfaden oder von zumindest einem Zusatzventil in einem der zwei Zusatz-Strömungspfade des Dialysierflüssigkeitskreislaufs, über welche ein Dialysierflüssigkeitszulauf und ein Dialysierflüssigkeitsablauf des Dialysierflüssigkeitskreislaufs fluidisch verbindbar sind, und zwar bei einem gleichzeitigen Einstellen, insbesondere einem gleichzeitigen Schließen, des Zulaufventils und des Ablaufventils, über die Steuereinheit auf. Dieser Schritt kann jederzeit nach dem Einstellen des Transmembrandrucks mit einem Druckgefälle von der Dialysierflüssigkeitsseite zur Blutseite des Dialysators bzw. nach dem Einstellen eines Transmembrandrucks mit einem Druckgefälle von der Blutseite zur Dialysierflüssigkeitsseite des Dialysators durchgeführt werden.

Das offenbarungsgemäße Verfahren ist abgeschlossen bzw. beendet, bevor ein Patient an den extrakorporalen Blutkreislauf angeschlossen wird. Das offenbarungsgemäße Verfahren ist somit kein Verfahren zur chirurgischen oder therapeutischen Behandlung des menschlichen oder tierischen Körpers und auch kein Diagnostizierverfahren, das am menschlichen oder tierischen Körper vorgenommen wird.

Die Offenbarung betrifft weiterhin ein Computerprogramm, umfassend Befehle, die bei einer Ausführung durch einen Computer diesen veranlassen, die folgenden Verfahrensschritte, bevorzugt die voranstehend beschriebenen Verfahrensschritte, auszuführen: Befüllen der Blutseite mit einer Flüssigkeit, insbesondere Priming-Flüssigkeit; Befüllen der Dialysierflüssigkeitsseite mit der Flüssigkeit, insbesondere Priming-Flüssigkeit; Einstellen eines Transmembrandrucks mit einem Druckgefälle von der Dialysierflüssigkeitsseite zur Blutseite des Dialysators über eine Steuereinheit, und Einstellen eines Transmembrandrucks mit einem Druckgefälle von der Blutseite zur Dialysierflüssigkeitsseite des Dialysators über die Steuereinheit durch ein Öffnen eines Ablaufventils und/oder ein Starten einer Pumpe in einem Dialysierflüssigkeitskreislauf, um Luft, insbesondere Luftblasen, beim Primen des Dialysators aus der semipermeablen Membran zu entfernen. Die Schritte Einstellen eines Transmembrandrucks mit einem Druckgefälle von der Dialysierflüssigkeitsseite zur Blutseite des Dialysators und Einstellen eines Transmembrandrucks mit einem Druckgefälle von der Blutseite zur Dialysierflüssigkeitsseite des Dialysators werden dabei nacheinander durchgeführt oder alternierend wiederholt. Dabei kann die Reihenfolge auch umgekehrt werden.

### Kurzbeschreibung der Figuren

Die Offenbarung wird nachfolgend anhand vorteilhafter Ausführungsformen und unter Bezugnahme auf die dazugehörenden Figuren näher erläutert. Es zeigen:
Fig. 1 eine vergrößerte isometrische Querschnittansichtsdarstellung zweier nebeneinander angeordneter Hohlfasern eines Hohlfaserdialysators der extrakorporalen Blutbehandlungsmaschine gemäß einer bevorzugten Ausführungsform der vorliegenden Offenbarung;
Fig. 2 eine vergrößerte Seitenschnittansicht der zwei in Fig.1 dargestellten nebeneinander angeordneten Hohlfasern eines Hohlfaserdialysators der extrakorporalen Blutbehandlungsmaschine gemäß der bevorzugten Ausführungsform der vorliegenden Offenbarung;
Fig. 3a eine schematische Seitenschnittansicht einer von einer Membran getrennten Blutseite und einer Dialysierflüssigkeitsseite eines Dialysators der extrakorporalen Blutbehandlungsmaschine gemäß der bevorzugten Ausführungsform der Offenbarung, mit einem Transmembrandruck mit einem Druckgefälle von der Blutseite zur Dialysierflüssigkeitsseite;
Fig. 3b eine schematische Seitenschnittansicht der in der Fig. 3a dargestellten Membran mit einem Transmembrandruck mit einem Druckgefälle von der Dialysierflüssigkeitsseite zur Blutseite;
Fig. 4 eine schematische Seitenschnittansicht durch eine Hohlfaser des Dialysators der extrakorporalen Blutbehandlungsmaschine gemäß der bevorzugten Ausführungsform der Offenbarung, mit einer angedeuteten laminaren und einer turbulenten Strömung einer Priming-Flüssigkeit auf der Blutseite;
Fig. 5 eine schematische Teilansicht der extrakorporalen Blutbehandlungsmaschine gemäß einer besonders bevorzugten Ausführungsform der Offenbarung; und
Fig. 6 ein Flussdiagramm eines automatischen Priming-Verfahrens gemäß einer bevorzugten Ausführungsform der vorliegenden Offenbarung.

Die Figuren sind lediglich schematischer Natur und dienen ausschließlich dem Verständnis der vorliegenden Offenbarung. Es wird darauf hingewiesen, dass die Merkmale der einzelnen Ausführungsformen untereinander ausgetauscht werden sowie in einer bestimmten Kombination auftreten können.

### Detaillierte Beschreibung bevorzugter Ausführungsformen

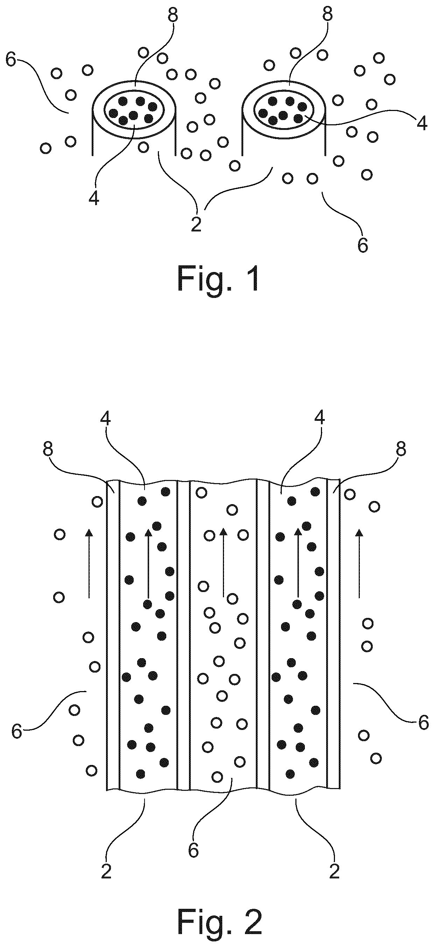

Nachfolgend wird die vorliegende Offenbarung anhand der bevorzugten Ausführungsformen mit Bezug auf die Figuren 1 bis 6 beschrieben.

Figur 1 zeigt eine vergrößerte isometrische Quer-Schnittansichtsdarstellung zweier nebeneinander angeordneter Hohlfasern 2 eines Hohlfaserdialysators der extrakorporalen Blutbehandlungsmaschine gemäß einer bevorzugten Ausführungsform der vorliegenden Offenbarung. Die Hohlfasern 2 verlaufen parallel zueinander und weisen eine innere Blutseite 4 sowie eine äußere Dialysierflüssigkeitsseite 6 auf. Die Blutseite 4 und die Dialysierflüssigkeitsseite 6 der jeweiligen Hohlfasern 2 sind durch eine semipermeable Membran 8 getrennt, welche in dieser Ausführungsform der Hohlfasern 2 schlauchförmig ausgebildet ist.

Figur 2 zeigt eine vergrößerte Seitenschnittansicht der zwei in Figur 1 dargestellten nebeneinander angeordneten Hohlfasern 2 des Hohlfaserdialysators der extrakorporalen Blutbehandlungsmaschine gemäß der bevorzugten Ausführungsform der vorliegenden Offenbarung. Deutlich zu erkennen sind die durch die Membranen 8 getrennten Blutseiten 4, die im Inneren der Hohlfasern 2 angeordnet sind, und die Dialysierflüssigkeitsseiten 6, die außerhalb der Hohlfasern 2 angeordnet sind. Ein bevorzugter Priming-Vorgang des Dialysators 19 wird dargestellt, bei welchem die Fließrichtungen der Priming-Flüssigkeiten auf der Blutseite 4 und der Dialysierflüssigkeitsseite 6 der jeweiligen Hohlfasern 2 die gleiche ist, um eventuelle Luftblasen im Dialysator 19 abzuführen. In Figur 2 verläuft diese Fließrichtung nach oben, also vorteilhafterweise in derselben Richtung, in welche Luftblasen innerhalb einer schwereren Flüssigkeit (entgegen der Schwerkraft) aufsteigen würden.

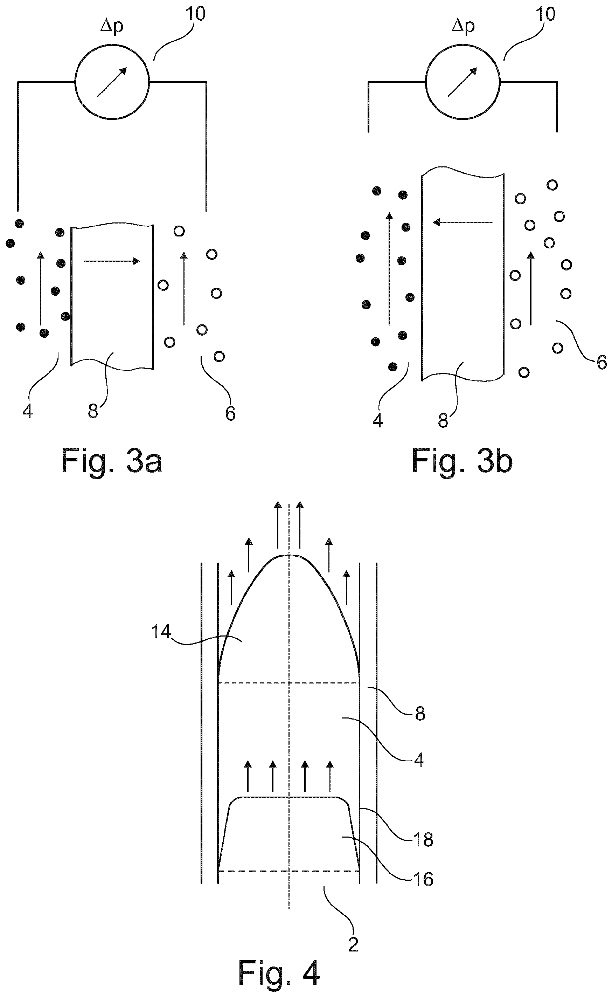

Figuren 3a und 3b zeigen jeweils eine schematische Seitenschnittansicht einer Blutseite 4 und einer Dialysierflüssigkeitsseite 6 eines Dialysators 19 der extrakorporalen Blutbehandlungsmaschine, die von der Membran 8 getrennt sind, gemäß der bevorzugten Ausführungsform der Offenbarung. Die Fließrichtungen der Priming-Flüssigkeiten auf beiden Seiten ist in diesen beiden Darstellungen ebenfalls dieselbe (hier dargestellt, nach oben). Dabei zeigt Figur 3a den Fall eines eingestellten Transmembrandrucks 10 mit einem Druckgefälle, d. h. einem Δp, von der Blutseite 4 zur Dialysierflüssigkeitsseite 6, also hier von links nach rechts. Figur 3b zeigt den Fall eines eingestellten Transmembrandrucks 10 mit einem Druckgefälle, d. h. einem Δp, von der Dialysierflüssigkeitsseite 6 zur Blutseite 4, also hier von rechts nach links. In beiden Fällen wird durch die Einstellung der seitlichen Durchströmung der Membran 8 sichergestellt, dass Luft bzw. Luftblasen aus der Membran 8 abgeführt werden und anschließend von der (Priming-)Flüssigkeit auf der Seite mit dem derzeitig geringeren Druck, vorzugsweise entgegen der Schwerkraft, abführbar sind.

Figur 4 zeigt eine schematische Seitenschnittansicht durch eine Hohlfaser 2 des Dialysators 19 der extrakorporalen Blutbehandlungsmaschine gemäß der bevorzugten Ausführungsform der Offenbarung, mit einer angedeuteten laminaren Strömung 14 und einer turbulenten Strömung 16 einer Priming-Flüssigkeit auf der Blutseite 4, also dem Inneren einer Hohlfaser 2 des Dialysators 19 (hier ebenfalls mit einer angedeuteten Fließrichtung nach oben). Deutlich zu erkennen ist eine dünnere (Strömungs-) Randschicht im Fall der turbulenten Strömung 16 im Bereich der Hohlfaser-Innenwand 18, wodurch ein gleichmäßigerer Strömungsverlauf der Flüssigkeit sichergestellt werden kann. Dies gewährleistet verbesserte Strömungsbedingungen um Luftblasen effizienter zu entfernen/ abzuführen.

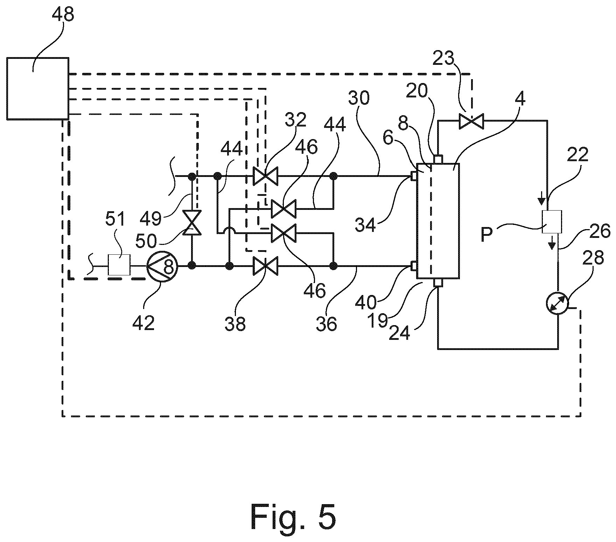

Figur 5 zeigt eine schematische Teilansicht der extrakorporalen Blutbehandlungsmaschine gemäß einer besonders bevorzugten Ausführungsform der Offenbarung. Dargestellt ist ein extrakorporaler Blutkreislauf mit einem über einen ersten blutseitigen Anschluss 20 mit dem Dialysator 19 verbundenen venösen Blutschlauchabschnitt 22, der wiederum ein venöses Absperrventil 23 oder eine venöse Schlauchklemme aufweist, sowie einem über einen zweiten blutseitigen Anschluss 24 mit dem Dialysator 19 verbundenen arteriellen Blutschlauchabschnitt 26. Beide Blutschlauchabschnitte 22, 26 sind beim Priming- Vorgang mit einem gemeinsamen (Waste-)Port/ Beutel P verbunden. Im arteriellen Blutschlauchabschnitt 26 ist vorteilhafterweise eine Blutpumpe 28 angeordnet, welche den Blutstrom im extrakorporalen Blutkreislauf antreibt. Der extrakorporale Blutkreislauf verläuft durch die Blutseite 4 des Dialysators, die durch die semipermeable Membran 8 von der Dialysierflüssigkeitsseite 6 des Dialysators 19 getrennt ist. Ein über die Dialysierflüssigkeitsseite 6 des Dialysators 19 verlaufender Dialysierflüssigkeitskreislauf läuft entweder in einer Hauptschluss-Schaltung über einen Dialysierflüssigkeitszulauf 30, welcher ein Zulaufventil 32 aufweist und über einen ersten dialysierflüssigkeitseitigen Anschluss 34 mit dem Dialysator 19 verbunden ist, und über einen Dialysierflüssigkeitsablauf 36, welcher ein Ablaufventil 38 aufweist und über einen zweiten dialysierflüssigkeitseitigen Anschluss 40 mit dem Dialysator 19 verbunden ist. Eine dem Ablaufventil 38 nachgeschaltete Pumpe 42 treibt den Strom der Dialysierflüssigkeit während der Therapie bzw. die (Priming-)Flüssigkeit auf der Dialysierflüssigkeitsseite 6 des Dialysators 19 während des Primens/ Priming-Vorgangs an. Bevorzugt sind der Pumpe 42 Bilanzkammern 51 nachgeschalten. Über ein Bypass-Strömungspfad 49 mit einem Bypass-Ventil 50 ist der Dialysierflüssigkeitszulauf 30 mit dem Dialysierflüssigkeitsablauf 36 fluidisch verbindbar/trennbar und ermöglicht das Umschalten des Dialysierflüssigkeitskreislaufs in eine Bypass-Schaltung (durch Schließen der vier Ventile 32, 38, 46, 46 und Öffnen des Ventils 50). In einem derartigen Bypass-Betrieb kann zunächst ein identischer Druck auf beiden Seiten der Membran 8 eingestellt werden und ein evtl. erzeugter Druckunterschied/ Transmembrandruck 10 zur Beseitigung von Luftblasen aus den Membran(en) 8 überträgt sich besser über die Membran(en) 8 ins komplette Gehäuse des Dialysators (bis zu den geschlossenen Ventilen 32, 38, 46, 46), also in die Dialysierflüssigkeitsseite 6 des Dialysators 19. In dieser bevorzugten Ausführungsform können bspw. durch ein (evtl. pulsartiges/abwechselndes) Öffnen/Schließen des Ablaufventils 38 und/oder des venösen Absperrventils 23/ der venösen Schlauchklemme, bevorzugt jeweils ausgehend von dem Bypass-Zustand identischer Drücke auf beiden Seiten der Membran 8, Druckimpulse in beide Richtungen durch die Membran 8 des Dialysators erzeugt werden, um Luftblasen aus dieser in die jeweilige Richtung abzuführen.

Zwei Zusatz-Strömungspfade 44, die jeweils ein Zusatzventil 46 aufweisen und über welche der Dialysierflüssigkeitszulauf 30 mit dem Dialysierflüssigkeitsablauf 36 fluidisch verbindbar/trennbar ist, ermöglichen das Umkehren der Fließrichtung des Dialysierflüssigkeitskreislaufs. Durch das Öffnen beider Zusatz-Ventile 46 und dem gleichzeitigen Schließen des Ablaufventils 38 und des Zulaufventils 32 sind diese über die Zusatz-Strömungspfade 44 umströmbar, wodurch eine automatische Umkehrung der Fließrichtung im Dialysierflüssigkeitskreislauf ermöglicht werden kann. Vorteilhafterweise kann die Fließrichtung der (Priming-) Flüssigkeiten auf der Blutseite 4 sowie auf der Dialysierflüssigkeitsseite 6 des Dialysators 19 gleichgerichtet werden. Bevorzugt befinden sich im montierten Zustand des Dialysators 19 an der Blutbehandlungsmaschine die Anschlüsse 24 und 40 in Schwerkraftrichtung weiter unten (näher an dem Boden, auf welchem die Blutbehandlungsmaschine steht) und befinden sich die Anschlüsse 20 und 34 in Schwerkraftrichtung weiter oben (weiter entfernt von dem Boden, auf welchem die Blutbehandlungsmaschine steht). Die Steuereinheit ist eingerichtet, beim Primen, die Ventile 32 und 38 zu schließen und die beiden Ventile 46 zu öffnen, um somit blutseitig und dialysierflüssigkeitsseitig identische Fließrichtungen im Dialysator, von unten nach oben, einzustellen, so dass der Dialysator während des Primens nicht von einem Operator gedreht werden muss.

Des Weiteren kommuniziert eine Steuereinheit 48 mit der Blutpumpe 28, der Ultrafiltrationspumpe 42, dem Zulaufventil 32, dem Ablaufventil 38, dem venösen Absperrventil 23/ der venösen Schlauchklemme, den Zusatz-Ventilen 46, und dem Bypass-Ventil 50, um Transmembrandrücke 10 im Dialysator 19, vorzugsweise alternierend, erzeugen zu können.

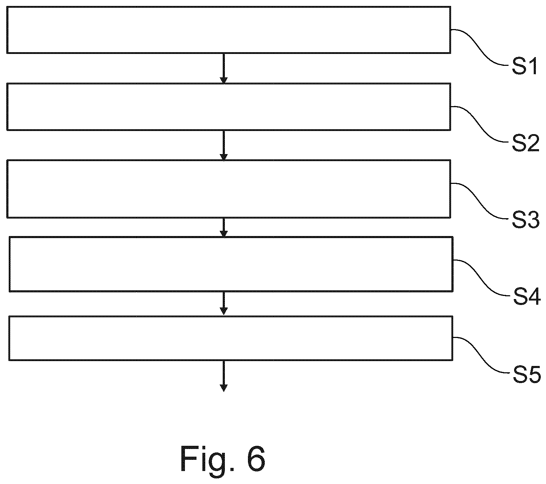

Figur 6 zeigt ein Flussdiagramm eines automatischen Priming-Verfahrens gemäß einer bevorzugten Ausführungsform der vorliegenden Offenbarung. Das Verfahren umfasst die Schritte Befüllen S1 der Blutseite 4 mit einer Flüssigkeit, insbesondere Priming-Flüssigkeit; Befüllen der Dialysierflüssigkeitsseite 6 mit der Flüssigkeit, insbesondere Priming-Flüssigkeit; Einstellen S3 eines Transmembrandrucks 10 mit einem Druckgefälle von der Dialysierflüssigkeitsseite 6 zur Blutseite 4 des Dialysators 19 über eine Steuereinheit 48; und Einstellen S4 eines Transmembrandrucks 10 mit einem Druckgefälle von der Blutseite 4 zur Dialysierflüssigkeitsseite 6 des Dialysators 19 über die Steuereinheit 48, um Luft, insbesondere Luftblasen, beim Primen des Dialysators 19 aus der semipermeablen Membran 8 zu entfernen. Die Schritte S3 Einstellen des Transmembrandrucks mit einem Druckgefälle von der Dialysierflüssigkeitsseite 6 zur Blutseite 4 des Dialysators 19 und S4 Einstellen eines Transmembrandrucks mit einem Druckgefälle von der Blutseite 4 zur Dialysierflüssigkeitsseite 6 des Dialysators 19 erfolgen dabei nacheinander oder (beliebig oft) alternierend wiederholend. In einem weiteren optionalen Schritt S5 kann die Flussrichtung auf der Dialysierflüssigkeitsseite 6 durch ein Einstellen von jeweils einem Zusatzventil 46 in zwei Zusatz-Strömungspfaden 44 des Dialysierflüssigkeitskreislaufs durch die Steuereinheit 48 umgekehrt werden, über welche ein Dialysierflüssigkeitszulauf 30 und ein Dialysierflüssigkeitsablauf 36 des Dialysierflüssigkeitskreislaufs fluidisch verbindbar sind. Dieser Schritt kann jederzeit nach dem Schritt S3 Einstellen des Transmembrandrucks mit einem Druckgefälle von der Dialysierflüssigkeitsseite 6 zur Blutseite 4 des Dialysators 19 bzw. nach dem Schritt S4 Einstellen eines Transmembrandrucks mit einem Druckgefälle von der Blutseite 4 zur Dialysierflüssigkeitsseite 6 des Dialysators 19 durchgeführt werden.

Das Verfahren kann die weiteren Schritte Einstellen einer Förderrate der Ultrafiltrationspumpe 42 über die Steuereinheit 48, sodass eine turbulente Strömung 16 auf der Dialysierflüssigkeitsseite 6 des Dialysators 19 erzeugt wird, und/oder Einstellen einer Förderrate der Blutpumpe 28 über die Steuereinheit 48, sodass eine turbulente Strömung 16 auf der Blutseite 4 des Dialysators 19 erzeugt wird, aufweisen. Diese Schritte sind dabei miteinander austauschbar oder können alternativ ganz weggelassen werden.

### Bezugszeichenliste

- 2: Hohlfaser
- 4: Blutseite
- 6: Dialysierflüssigkeitsseite
- 8: Membran
- 10: Transmembrandruck
- 14: laminare Strömung
- 16: turbulente Strömung
- 18: Hohlfaser-Innenwand
- 19: Dialysator
- 20: erster blutseitiger Anschluss
- 22: venöser Blutschlauchabschnitt
- 23: venöses Absperrventil
- 24: zweiter blutseitiger Anschluss
- 26: arterieller Blutschlauchabschnitt
- 28: Blutpumpe
- 30: Dialysierflüssigkeitszulauf
- 32: Zulaufventil
- 34: erster dialysierflüssigkeitseitiger Anschluss
- 36: Dialysierflüssigkeitsablauf
- 38: Ablaufventil
- 40: zweiter dialysierflüssigkeitseitiger Anschluss
- 42: Pumpe/ Ultrafiltrationspumpe
- 44: Zusatz-Strömungspfade
- 46: Zusatzventile
- 48: Steuereinheit
- 49: Bypass-Strömungspfad
- 50: Bypass-Ventil
- 51: Bilanzkammer(n)

- P: (Waste-)Port
- S1: Schritt Befüllen der Blutseite mit einer Flüssigkeit
- S2: Schritt Befüllen der Dialysierflüssigkeitsseite mit einer Flüssigkeit
- S3: Einstellen eines Transmembrandrucks mit einem Druckgefälle von der Dialysierflüssigkeitsseite zur Blutseite
- S4: Einstellen eines Transmembrandrucks mit einem Druckgefälle von der Blutseite zur Dialysierflüssigkeitsseite
- S5: Umkehren einer Flussrichtung auf der Dialysierflüssigkeitsseite

## Patentansprüche

1. Extrakorporale Blutbehandlungsmaschine, insbesondere Dialysemaschine, aufweisend:
- einen Hohlfasern (2) aufweisenden Dialysator (19) mit einer Blutseite (4) und einer Dialysierflüssigkeitsseite (6), welche voneinander durch eine semipermeable Membran (8) getrennt sind,
- einen extrakorporalen Blutkreislauf, welcher über einen ersten blutseitigen Anschluss und einen zweiten blutseitigen Anschluss des Dialysators (19) durch die Blutseite (4) des Dialysators (19) verläuft,
- einen Dialysierflüssigkeitskreislauf, welcher über einen ersten dialysierflüssigkeitseitigen Anschluss und einen zweiten dialysierflüssigkeitseitigen Anschluss des Dialysators (19) durch die Dialysierflüssigkeitsseite (6) des Dialysators (19) verläuft,
**gekennzeichnet durch** eine Steuereinheit (48), welche eingerichtet und vorbereitet ist, ein automatisches Primen der Blutbehandlungsmaschine derart durchzuführen, dass sie nacheinander oder alternierend einen Transmembrandruck (10) mit einem Druckgefälle von der Dialysierflüssigkeitsseite (6) zur Blutseite (4) und von der Blutseite (4) zur Dialysierflüssigkeitsseite (6) einstellt, um Luft, insbesondere Luftblasen, beim Primen aus der semipermeablen Membran (8) zu entfernen, wobei der Dialysierflüssigkeitskreislauf einen Dialysierflüssigkeitszulauf (30) mit einem Zulaufventil (32), einen Dialysierflüssigkeitsablauf (36) mit einem Ablaufventil (38) sowie eine Pumpe (42) aufweist, und die Steuereinheit (48) eingerichtet und vorbereitet ist, den Transmembrandruck (10) mit einem Druckgefälle von der Blutseite (4) zur Dialysierflüssigkeitsseite (6) durch ein Öffnen des Ablaufventils (38) und/oder durch ein Betätigen der Pumpe (42) einzustellen.

2. Extrakorporale Blutbehandlungsmaschine nach Anspruch 1, **dadurch gekennzeichnet, dass** der extrakorporale Blutkreislauf einen arteriellen Blutschlauchabschnitt (26) mit einer Blutpumpe (28) und einen venösen Blutschlauchabschnitt (22) mit einer venösen Absperreinrichtung, beispielsweise ein venöses Absperrventil (23) oder eine venöse Schlauchklemme, aufweist, und die Steuereinheit (48) eingerichtet und vorbereitet ist, den Transmembrandruck (10) mit einem Druckgefälle von der Blutseite (4) zur Dialysierflüssigkeitsseite (6) über die Blutpumpe (28) und/oder ein Schließen der venösen Absperreinrichtung einzustellen, und/oder den Transmembrandruck (10) mit einem Druckgefälle von der Dialysierflüssigkeitsseite (6) zur Blutseite (4) durch ein Öffnen der venösen Absperreinrichtung einzustellen.

3. Extrakorporale Blutbehandlungsmaschine nach einem der vorhergehenden Ansprüche, **dadurch gekennzeichnet, dass** die Steuereinheit (48) eingerichtet ist, den Transmembrandruck (10) alternierend mit einem Druckgefälle von der Dialysierflüssigkeitsseite (6) zur Blutseite (4) und von der Blutseite (4) zur Dialysierflüssigkeitsseite (6) einzustellen.

4. Extrakorporale Blutbehandlungsmaschine nach einem der vorhergehenden Ansprüche, **dadurch gekennzeichnet, dass** der Dialysierflüssigkeitskreislauf einen Bypass-Pfad (49) mit einem Bypass-Ventil (50) aufweist, über welches ein Bypass-Zustand der Blutbehandlungsmaschine einstellbar ist, und die Steuereinheit (48) eingerichtet und vorbereitet ist, ausgehend von diesem Bypass-Zustand, den Transmembrandruck (10) mit einem Druckgefälle von der Dialysierflüssigkeitsseite (6) zur Blutseite (4) und von der Blutseite (4) zur Dialysierflüssigkeitsseite (6) durch ein gegensätzliches Öffnen und Schließen der venösen Absperreinrichtung sowie des Ablaufventils (38) alternierend einzustellen.

5. Extrakorporale Blutbehandlungsmaschine nach einem der vorhergehenden Ansprüche, **dadurch gekennzeichnet, dass** die Steuereinheit (48) eingerichtet ist, den Transmembrandruck (10) mit einem Druckgefälle von der Dialysierflüssigkeitsseite (6) zur Blutseite (4) und/oder von der Blutseite (4) zur Dialysierflüssigkeitsseite (6) jeweils pulsierend einzustellen, um Druckstöße von der Dialysierflüssigkeitsseite (6) zur Blutseite (4) und/oder von der Blutseite (4) zur Dialysierflüssigkeitsseite (6) zu erzeugen.

6. Extrakorporale Blutbehandlungsmaschine nach einem der vorhergehenden Ansprüche, **dadurch gekennzeichnet, dass** der Dialysierflüssigkeitskreislauf zumindest zwei Zusatz-Strömungspfade (44) mit jeweils einem Zusatzventil (46) aufweist, über welche der Dialysierflüssigkeitszulauf (30) und der Dialysierflüssigkeitsablauf (36) des Dialysierflüssigkeitskreislaufs fluidisch verbindbar sind, und die Steuereinheit (48) eingerichtet ist, die Zusatzventile (46) beim Primen derart zu steuern, dass eine Flussrichtung auf der Dialysierflüssigkeitsseite (6) umkehrbar ist.

7. Verfahren zum automatischen Primen eines Hohlfasern (2) aufweisenden Dialysators (19) einer extrakorporalen Blutbehandlungsmaschine, mit einer Blutseite (4) und einer Dialysierflüssigkeitsseite (6), welche voneinander durch eine semipermeable Membran (8) getrennt sind, mit den Schritten:
- Befüllen (S1) der Blutseite (4) mit einer Flüssigkeit, insbesondere Priming-Flüssigkeit;
- Befüllen (S2) der Dialysierflüssigkeitsseite (6) mit der Flüssigkeit, insbesondere Priming-Flüssigkeit;
- Einstellen (S3) eines Transmembrandrucks (10) mit einem Druckgefälle von der Dialysierflüssigkeitsseite (6) zur Blutseite (4) des Dialysators (19) über eine Steuereinheit (48); und
- Einstellen (S4) eines Transmembrandrucks (10) mit einem Druckgefälle von der Blutseite (4) zur Dialysierflüssigkeitsseite (6) des Dialysators (19) über die Steuereinheit (48) durch ein Öffnen eines Ablaufventils (38) und/oder ein Starten einer Pumpe (42) in einem Dialysierflüssigkeitskreislauf,
um Luft, insbesondere Luftblasen, beim Primen des Dialysators (19) aus der semipermeablen Membran (8) zu entfernen, wobei die Schritte Einstellen (S3) eines Transmembrandrucks (10) mit einem Druckgefälle von der Dialysierflüssigkeitsseite (6) zur Blutseite (4) des Dialysators (19) und Einstellen (S4) eines Transmembrandrucks (10) mit einem Druckgefälle von der Blutseite (4) zur Dialysierflüssigkeitsseite (6) des Dialysators (19) nacheinander durchgeführt oder alternierend wiederholt werden.

8. Verfahren nach Anspruch 7, **dadurch gekennzeichnet, dass** der Schritt Einstellen (S3) des Transmembrandrucks (10) mit dem Druckgefälle von der Dialysierflüssigkeitsseite (6) zur Blutseite (4) durch ein Öffnen einer venösen Absperreinrichtung in einem extrakorporalen Blutkreislauf durchgeführt wird.

9. Verfahren nach Anspruch 7 oder 8, **dadurch gekennzeichnet, dass** der Schritt Einstellen (S4) des Transmembrandrucks (10) mit dem Druckgefälle von der Blutseite (4) zur Dialysierflüssigkeitsseite (6) des Dialysators (19) durch ein Starten zumindest einer Blutpumpe (28) und/oder ein Schließen der venösen Absperreinrichtung im extrakorporalen Blutkreislauf und durch ein Öffnen des Ablaufventils (38) und/oder ein Starten der Pumpe (42) im Dialysierflüssigkeitskreislauf durchgeführt wird.

10. Verfahren nach einem der Ansprüche 7 bis 9, **dadurch gekennzeichnet, dass** die Schritte Einstellen (S3) eines Transmembrandrucks (10) mit einem Druckgefälle von der Dialysierflüssigkeitsseite (6) zur Blutseite (4) des Dialysators (19) und Einstellen (S4) eines Transmembrandrucks (10) mit einem Druckgefälle von der Blutseite (4) zur Dialysierflüssigkeitsseite (6) des Dialysators (19) alternierend wiederholt werden.

11. Verfahren nach Anspruch 7, **dadurch gekennzeichnet, dass**, ausgehend von einem Bypass-Zustand der extrakorporalen Blutbehandlungsmaschine, die Schritte Einstellen (S3) des Transmembrandrucks (10) mit einem Druckgefälle von der Dialysierflüssigkeitsseite (6) zur Blutseite (4) und Einstellen (S4) des Transmembrandrucks (10) mit einem Druckgefälle von der Blutseite (4) zur Dialysierflüssigkeitsseite (6) durch ein gegensätzliches Öffnen und Schließen einer venösen Absperreinrichtung im extrakorporalen Blutkreislauf sowie eines Ablaufventils (38) im Dialysierflüssigkeitskreislauf alternierend wiederholt werden.

12. Verfahren nach einem der Ansprüche 7 bis 11, **dadurch gekennzeichnet, dass** die Schritte Einstellen (S3) des Transmembrandrucks (10) mit einem Druckgefälle von der Dialysierflüssigkeitsseite (6) zur Blutseite (4) des Dialysators (19) und Einstellen (S4) des Transmembrandrucks (10) mit einem Druckgefälle von der Blutseite (4) zur Dialysierflüssigkeitsseite (6) des Dialysators (19) jeweils pulsierend durchgeführt werden, um Druckstöße von der Dialysierflüssigkeitsseite (6) zur Blutseite (4) und/oder von der Blutseite (4) zur Dialysierflüssigkeitsseite (6) zu erzeugen.

13. Verfahren nach einem der Ansprüche 7 bis 12, **gekennzeichnet durch** einen zusätzlichen Schritt Umkehren (S5) einer Flussrichtung auf der Dialysierflüssigkeitsseite (6) durch ein Einstellen von jeweils einem Zusatzventil (46) in zwei Zusatz-Strömungspfaden (44) des Dialysierflüssigkeitskreislaufs, über welche ein Dialysierflüssigkeitszulauf (30) und ein Dialysierflüssigkeitsablauf (36) des Dialysierflüssigkeitskreislaufs fluidisch verbindbar sind, bei einem gleichzeitigen Einstellen des Zulaufventils (32) und des Ablaufventils (38), über die Steuereinheit (48).

14. Computerprogramm, umfassend Befehle, die bei einer Ausführung durch einen Computer diesen veranlassen, die folgenden Verfahrensschritte, insbesondere gemäß dem Verfahren nach einem der Ansprüche 7 bis 13, auszuführen:
Befüllen (S1) der Blutseite (4) mit einer Flüssigkeit, insbesondere Priming-Flüssigkeit;
- Befüllen (S2) der Dialysierflüssigkeitsseite (6) mit der Flüssigkeit, insbesondere Priming-Flüssigkeit;
- Einstellen (S3) eines Transmembrandrucks (10) mit einem Druckgefälle von der Dialysierflüssigkeitsseite (6) zur Blutseite (4) des Dialysators (19) über eine Steuereinheit (48), und
- Einstellen (S4) eines Transmembrandrucks (10) mit einem Druckgefälle von der Blutseite (4) zur Dialysierflüssigkeitsseite (6) des Dialysators (19) über die Steuereinheit (48) durch ein Öffnen eines Ablaufventils (38) und/oder ein Starten einer Pumpe (42) in einem Dialysierflüssigkeitskreislauf,
um Luft, insbesondere Luftblasen, beim Primen des Dialysators (19) aus der semipermeablen Membran (8) zu entfernen, wobei die Schritte Einstellen (S3) eines Transmembrandrucks (10) mit einem Druckgefälle von der Dialysierflüssigkeitsseite (6) zur Blutseite (4) des Dialysators (19) und Einstellen (S4) eines Transmembrandrucks (10) mit einem Druckgefälle von der Blutseite (4) zur Dialysierflüssigkeitsseite (6) des Dialysators (19) nacheinander durchgeführt oder alternierend wiederholt werden.
